# EUROPEAN PATENT APPLICATION

(11) **EP 1 671 753 A1**
(43) Date of publication of application: **21.06.2006**
(21) Application number: 03818667.2
(22) Date of filing: 12.09.2003
(51) Int. Cl.: B25J 5/00, B25J 19/00

(54) **DIRECT-ACTING LINK DEVICE AND TWO-LEGGED WALKING ROBOT WITH THE DEVICE**

(71) Applicant: TMSUK Co., Ltd., Kitakyushu-shi, Fukuoka 803-0851 (JP); Takanishi, Atsuo, Tokyo 165-0024 (JP)
(72) Inventor: Takanishi, Atsuo, Nakano-ku, Tokyo 165-0024 (JP); Ino, Shigeaki, Tmsuk Co., Ltd., Fukuoka 803-0851 (JP); Takamoto, Yoichi, Tmsuk Co., Ltd., Fukuoka 803-0851 (JP); Baba, Katsuyuki, Tmsuk Co., Ltd., Fukuoka 803-0851 (JP)
(74) Representative: HOFFMANN EITLE
(86) International application number: PCT/JP2003/011699
(87) International publication number: WO 2005/025815

(57) **Abstract**

It is an object of the present invention to provide a linear motion link device capable of reducing a load applied to an actuator and capable of saving consumption of power and a bipedal walking robot provided therewith. Therefore, this is why the linear motion link device is held and locked by a holding brake so as not to telescope during the stop of the actuator. The linearmotion link device of the present invention is constituted so as to include an actuator (28) and a holding brake (30) which holds and locks a movable portion (41) of the actuator (28) and to freely telescope in a longitudinal direction of the linear motion link device (1) by driving of the actuator (28). Further, a bipedal walking robot of the present invention has a constitution that the linear motion link device (1) having the holding brake (30) which holds and locks the movable portion (41) of the actuator (28) is provided.

## Description

### Technical Field

The present invention relates to a linear motion link device capable of being held and locked by a holding brake so as not to telescope during the stop of an actuator and a bipedal walking robot provided therewith.

### Background Art

Conventionally, various constitutions regarding a robot employing a parallel link mechanism have been proposed. A typical parallel link mechanism includes six linearmotion links telescope-driven, and has a constitution in which an end of each linear motion link is connected to a base via a universal joint and the other end thereof is connected to an end effector via other universal joint.

As a robot employing such a parallel link mechanism, "a parallel link manipulator comprising a base plate, an end effector and six linear motion links which respectively connect the base plate with the end effector and respectively have an actuator, in which the linear motion link has a universal joint on an end and has a universal joint and a rotary joint around a shaft connected in series to the universal joint on the other end, and these linear motion links are arranged close to the shaft in line symmetry to a direction for desiring to exert out large power" is disclosed in Japanese Published Unexamined Patent Application No.7-060678 (referred to as "Patent reference 1" hereinafter).

However, in the above-described conventional art, there remain problems as described below.
(1) In the parallel link manipulator disclosed in Patent reference 1, the end effector is supported by the linear motion links, however, the linear motion links are required to support the weight of the end effector themselves and a weight applied to the end effector during not only operation but at standstill. Therefore, there is a problem that the actuators are required to drive, and a large load is applied to the actuators.
(2) Further, in a case that a robot employing the parallel link mechanism mounts a battery as a power source, the actuators are required to drive during not only operation but at standstill. Therefore, there is a problem that operating time by exchanging or charging of the battery at one time is shortened, and an operating rate of the robot lowers.

In order to solve the above-described conventional problems, the present invention was made, and it is an object of the present invention to provide a linear motion link device and a bipedal walking robot provided therewith capable of reducing a load applied to an actuator and capable of saving power consumption owing to a holding brake which can hold and lock the link device so that the link device does not telescope.

### Disclosure of the Invention

In order to solve the above-described problems, a linear motion link device and a bipedal walking robot provided therewith of the present invention have a constitution as described below.

A linear motion link device according to the first aspect of the invention is constituted so as to include an actuator and a holding brake which holds and locks a movable portion of the actuator, and to freely telescope in a longitudinal direction thereof by driving of the actuator.

By this constitution, the linear motion link device has operations as described below.
(1) Even if the actuator is stopped in a state that a heavy load is supported by the linear motion link device, the movable portion is held and locked by the holding brake. Therefore, the movable portion is not driven by a weight of the heavy load, the supporting state can be maintained.
(2) The actuator can be stopped in the state that the heavy load is supported by the linear motion link device, and therefore a load applied to the actuator can be reduced, and consumption of operating energy of the actuator can be saved.

Here, as the actuator, a motor driven by power, an oil hydraulic cylinder, a hydraulic cylinder, an air pressure cylinder or a linear motion actuator. etc., is employed.

Further, as the holding brake, when the actuator is a motor, etc., an object, which performs braking by frictional force owing to pressure of a pressing plate, is employed, and when the actuator is a cylinder, an opening/shutting valve or a servo valve, which stops feeding of oil or water, is employed. Furthermore, when the holding brake performs braking by frictional force owing to pressure of the pressing plate, in order to press-contact the pressing plate with a pressing plate braking portion, either both of them may be provided with a permanent magnet, be magnetized or be magnetically attracted, or the pressing plate may be press-contacted with the pressing plate braking portion by elastic force of an elastic body such as a spring, etc.

A linear motion link device according to the second aspect of the invention has a constitution that the holding brake holds and locks the movable portion of the actuator during the stop of the actuator in the invention according to the first aspect.

By this constitution, in addition to operation of the first aspect, the linear motion link device has an operation as described below.
(1) Even if the motor suddenly stops by a breakdown, etc., in the state that the heavy load is supported by the linear motion link device, the supporting state can be maintained by holding and locking the movable portion with use of the holding brake, and therefore it is excellent in stability and safety.

A linear motion link device according to the third aspect of the invention is constituted so as to include: in the invention according to the second aspect, a motor as the actuator; a long bar-shaped male screw shaft portion fixed to a rotary shaft of the motor and provided in the longitudinal direction of the linear motion link device; a female screw nut portion screw-engaged with the male screw shaft portion; an inner rod portion which is fixed to the female screw nut portion and slides in the longitudinal direction of the linear motion link device by driving of the motor; and a holding brake which holds and locks the rotary shaft of the motor.

By this constitution, in addition to operation of the second aspect, the linear motion link device has operations as described below.
(1) The inner rod portion can be locked by braking of rotating of the rotary shaft with use of the holding brake, and therefore the motor can be stopped in the state that the heavy load is supported by the linear motion link device, the supporting state can be maintained.
(2) When the linear motion link device stands still in the state of supporting the heavy load, the motor can be stopped with the standing still state maintained by the holding brake, and therefore a load torque applied to the motor can be reduced, and consumption power of the motor can be saved.

A bipedal walking robot according to the forth aspect of the invention is constituted so as to include: a base; a right foot and a left foot; a plurality of passive joints respectively provided on the base, the right foot and the left foot; parallel link mechanism portions respectively provided between the passive joint provided on the base and the passive joints provided on the right foot and between the passive joint provided on the base and the passive joints provided on the left foot; and the linear motion links according to any one of the first to third aspects as the link of the parallel link mechanism portion.

By this constitution, the bipedal walking robot has operations as described below.
(1) In a standing still state, that is, a posture fixed state of the bipedal walking robot, the movable portion of the actuator can be held and locked by the holding brake. Therefore, the movable portion of the actuator is not driven by a weight of the robot itself, the standing still state can be maintained, and a load applied to the actuator can be reduced.
(2) In the standing still state, that is, the posture fixed state of the bipedal walking robot, supplying operation energy to the actuator can be stopped, so that consumption energy of the actuator canbe reduced. For example, when power is supplied to the actuator by a mounted battery, operating time by exchanging or charging of the battery one time is extended and an operating rate of the bipedal walking robot can be improved.
(3) Even if the actuator suddenly stops by a breakdown, etc., during the operation, the bipedal walking robot can maintain the current posture. Therefore, neither damage of the bipedal walking robot itself by a fall nor an accident by a fall occur, and in this point, it is excellent in stability and safety.
(4) At the time of transporting or waiting of the bipedal walking robot, a posture of the bipedal walking robot can be maintained without driving of the actuator, and therefore a load torque applied to the actuator can be reduced, and consumption of operating energy of the actuator can be saved.

A bipedal walking robot according to the fifth aspect of the invention is constituted so as to include three sets of the parallel link mechanism portions which are provided between the base and the right foot and between the base and the left foot in the invention according to the forth aspect.

By this constitution, in addition to operation of the forth aspect, the bipedal walking robot has an operation as described below.
(1) Each parallel link mechanism portion employed in the right and left legs is constituted by a Stewart platform in which the two links as one set are provided in a V-shape and the three sets are provided, and therefore it is excellent in stability and rigidity, and operation controlling can be simplified.

### Brief Description of the Drawings

Fig. 1 is a perspective view of a main part of a linear motion link device of a first embodiment;
Fig. 2A is a side view of the main part of the linear motion link device of the first embodiment;
Fig. 2B is a cross sectional view of the main part of the linear motion link device taken along the A-A line of Fig. 2A and viewed from an arrow direction;
Fig. 3A is a constitutional view showing a state of a holding brake during the driving of a motor;
Fig. 3B is a constitutional view showing a state of a holding brake during the stop of the motor; and
Fig. 4 is a perspective view of a bipedal walking robot of a second embodiment.

### Best Mode of Carrying Out the Invention

Embodiments of the present invention will be explained below referring to the accompanying drawings.

### Embodiment 1

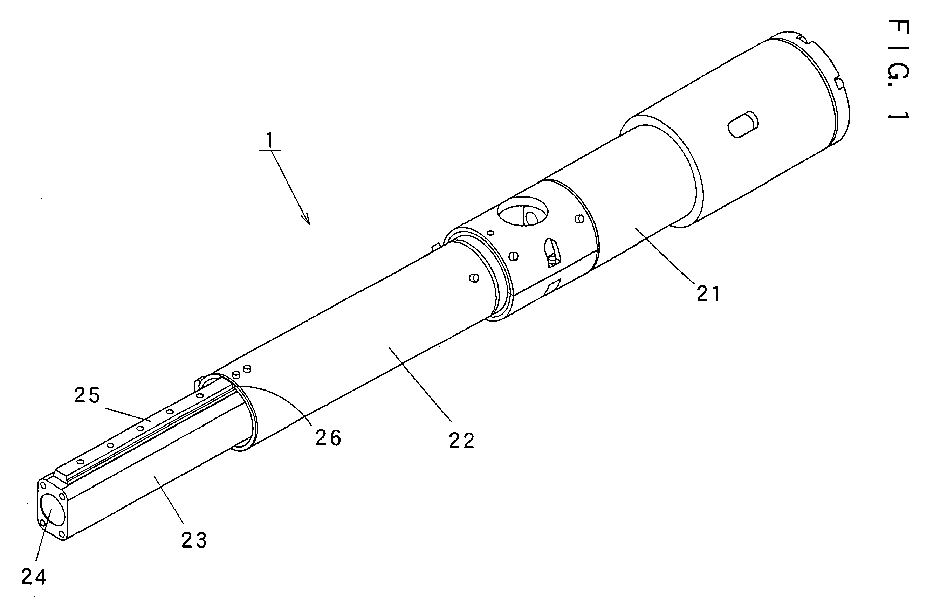

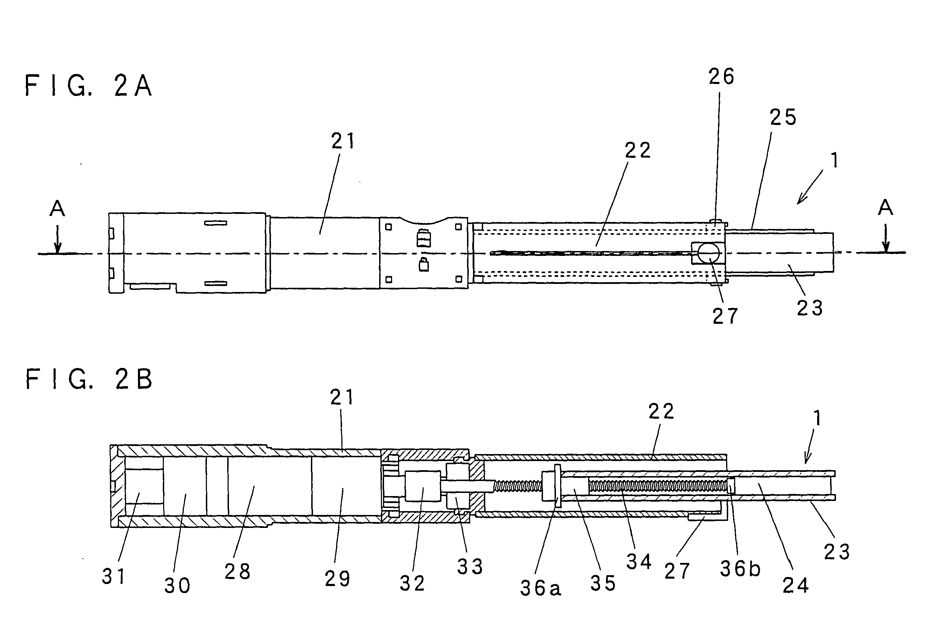

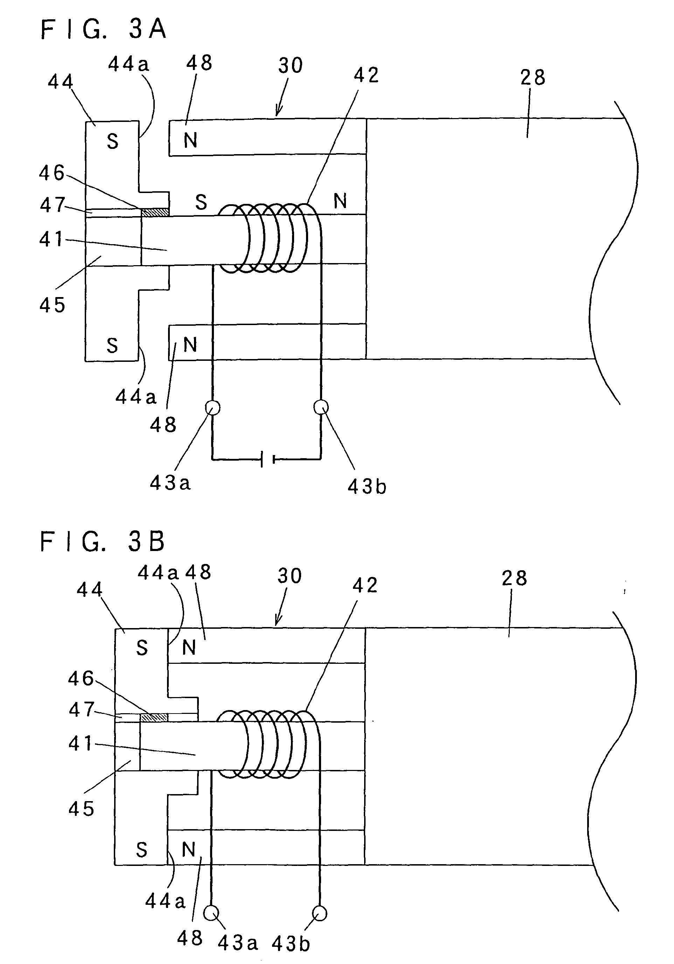

Fig. 1 is a perspective view of a main part of a linear motion link device of a first embodiment, Fig. 2A is a side view of the main part of the linear motion link device of the first embodiment, Fig. 2B is a cross sectional view of the main part of the linear motion link device taken along the A-A line of Fig. 2A and viewed from an arrow direction, Fig. 3A is a constitutional view showing a state of a holding brake during the driving of a motor and Fig. 3B is a constitutional view showing a state of a holding brake during the stop of the motor.

In Fig. 1 or Fig. 2, reference symbol 1 denotes a linear motion link device, 21 denotes a holding casing which holds a motor, a gear unit, a holding brake and a rotary encoder, etc., described below, 22 denotes a hollow outer tube portion extended from the holding casing 21 and 23 denotes an inner rod portion which is inserted into the outer tube portion 22 and slides in a longitudinal direction of the linear motion link device 1. Telescoping of the linear motion link device 1 is performed by sliding of the inner rod portion 23. Reference symbol 24 denotes a male screw shaft insertion hole formed in the inner rod portion 23 and in a longitudinal direction of the portion 23, 25 denotes a rod rail portion formed on an outer circumference of the inner rod portion 23 in the longitudinal direction of the inner rod portion 23, the rails vertically facing to each other, 26 denotes a rail guide portion with which the rod rails portion 25 is engaged to slide, 27 denotes an initial position sensor which detects an initial position of the inner rod portion 23, 28 denotes a motor as an actuator which makes the linear motion link device 1 telescope, 29 denotes a gear unit which reduces a rotating speed of a rotary shaft of the motor 28 to a predetermined rotating speed, 30 denotes a holding brake provided on a rear portion side of the motor 28, 31 denotes a rotary encoder which detects rotating of the rotary shaft of the motor 28, 32 denotes a coupling which connects the rotary shaft with a male screw shaft portion as described below via the gear unit 29, 33 denotes a bearing which supports the male screw shaft portion as described below, 34 denotes the male screw shaft portion which is inserted and provided into the male screw shaft insertion hole 24 in the inner rod portion 23 inside the outer tube portion 22, and whose outer circumference is screw-threaded, 35 denotes a female screw nut portion which is fixed in the male screw shaft insertion hole 24 in an end of the inner rod 23 inside the outer tube 22 and is screw-engaged with the mail screw shaft portion 34, and 36a and 36b respectively denote a stopper.

In Fig. 3, reference symbol 28 denotes the motor, 30 denotes the holding brake, 41 denotes the rotary shaft of the motor 28 extended to the rear portion side of the motor 28, 42 denotes a coil portion provided circumferentially on the rotary shaft 41 and 43a and 43b respectively denote a terminal of the coil portion 42. The terminals 43a and 43b of the coil portion 42 are connected to a logic circuit (not shown), the logic circuit applies or stops applying voltage to the coil portion 42 via a relay by an instruction from an outer controller such as a computer, etc. Thus, the coil portion 42 is energized or non-energized with driving or the stop of the motor 28. In place of the logic circuit, it may be controlled so that the terminals 43a and 43b are connected in parallel with a wire of a driving source connected to the motor 28 and voltage is applied to the coil portion 42 synchronizing with driving or the stop of the motor 28.

As a timing of applying voltage to the coil portion 42, for example, assuming that the motor 28 is in a state of a stop and voltage is not applied to the coil portion 42, when an instruction of speed for making the motor 28 rotate is transmitted from the outer controller to a motor driver which controls the motor 28, the logic circuit applies voltage to the coil portion 42 and releases the holding brake 30 from an holding and locking state with driving of the motor 28. As a timing of stopping applying voltage to the coil portion 42, for example, assuming that the motor 28 is driven and voltage is applied to the coil portion 42, when an instruction of speed for making a rotating speed of the motor 28 zero is transmitted from the outer controller to the motor driver which controls the motor 28, the logic circuit stops applying voltage to the coil portion 42 and makes the holding brake 30 operate with the stop of the motor 28.

Reference symbol 44 denotes a disc-shaped pressing plate slidably provided on the rotary shaft 41 in a longitudinal direction thereof, 44a denotes a pressing surface of the pressing plate 44 formed at a pressing plate braking portion side described below, 45 denotes a rotary shaft insertion hole which is bored in a center portion of the pressing plate 44 and into which the rotary shaft 41 is inserted, 4 6 denotes a key protruded on a tip of the rotary shaft 41, 47 denotes a key sliding groove which is formed on an inner circumference surface of the rotary shaft insertion hole 45 of the pressing plate 44 and with which the key 46 is engaged to slide and 48 denotes the pressing plate braking portion provided at a motor 28 side of the pressing plate 44 facing the pressing surface 44a.

Here, the pressing plate 44 and the pressing plate braking portion 48 are constituted to be magnetically attracted to each other. The pressing plate 44 is magnetized to the S pole or N pole at least in the pressing surface 44a, the pressing plate braking portion 48 provided facing the pressing surface 44a is magnetized to the N pole or S pole. Further, the pressing plate 44 and the pressing plate braking portion 48 may respectively employ a permanent magnetic magnetized to a predetermined magnetic pole, or a predetermined magnetic force may be generated by energizing to the pressing plate 44 and the pressing plate braking portion 48 which are wound with a coil, etc. Forexample, when the pressing plate 44 ismagnetized to the S pole and the pressing plate braking portion 48 is magnetized to the N pole, magnetic force generated by the coil portion 42 is established to the S pole to repel the S pole of the pressing plate 44. Further, magnetic force of the pressing plate braking plate 48 is established to become smaller than magnetic force generated by energizing the coil portion 42. At the time of energizing the coil portion 42, magnetic force of the S pole generated at a pressing plate 44 side of the coil portion 42 is larger than magnetic force of the N pole of the pressing plate braking portion 48, and therefore the pressing plate 44 is repelled to the rear portion side of the motor 28 and separated from the pressing plate braking portion 48.

Further, braking force of the holding brake 30 is in proportion to pressing force in a case that the pressing plate 44 is pressed to the pressing plate braking portion 48 . Pressing force of the pressing plate 44 to the pressing plate braking portion 48 is adjusted by properly establishing the magnetic force of the S pole or N pole of the pressing plate 44 or the pressing plate braking portion 48 and magnetic force generated by the coil portion 42. Especially, magnetic force generated by the coil portion 42 can be enlarged by enlarging voltage applied to the coil portion 42. Moreover, it is preferable that the braking force of the holding brake 30 is established considering a reducing speed ratio of the gear unit 29 or a lead angle between the male screw shaft portion 34 and the female screw nut portion 35 screw-engaged with the shaft portion 34, etc. Further, it is preferable that voltage applied to the coil portion 42 is established so that the holding brake 30 can obtain an established braking force.

Further, the pressing plate 44 rotates in accordance with rotating of the rotary shaft 41 and freely slides in the longitudinal direction of the rotary shaft 41, because the key 46 protruded on a circumference surface of the rotary shaft 41 is slidably engaged with the key sliding groove 47 formed in the rotary shaft insertion hole 45 of the pressing plate 44. Moreover, it is preferable that a stopper (not shown) is provided on at least an end of the motor 28 side of the key sliding groove 47 to prevent coming-off of the key 41.

The operation of the linear motion link device of the first embodiment constituted as described above will be explained with reference to the drawings.

As shown in Fig. 1 and Fig. 2, when the motor 28 is driven, the rotary shaft of the motor 28 rotates, the rotating speed is reduced with a predetermined reducing speed ratio by the gear unit 29, and the male screw shaft portion 34 rotates. The female screw nut portion 35 fixed to the inner rod portion 23 is screw-engaged with the male screw shaft portion 34. Here, the inner rod portion 23 slides in a longitudinal direction thereof, however, it does not rotate in the axis circumference direction thereof, because the rod rail portion 25 provided on the outer circumference surface of the inner rod portion 23 is engaged with the rail guide 26. Thus, when the male screw shaft portion 34 rotates, the inner rod portion 23 slides in the longitudinal direction thereof by the feed screw mechanism via the female screw nut portion 35, and the linear motion link device 1 telescopes.

As shown in Fig. 3A, voltage is applied to energize the coil portion 42 of the holding brake 30 during the driving of the motor 28. In the first embodiment, the voltage of 24V is applied to the coil portion 42 with the driving of the motor 28. When the coil portion 42 is energized, magnetic force of the S pole is generated at the pressing plate 44 side by the coil portion 42 to repel magnetic force of the S pole generated at the pressing plate 44 side of the coil portion 42, and the pressing plate 44 is urged toward the rear portion side of the motor 28. Thus, the pressing plate 44 is separated from the pressing plate braking portion 48 to rotate in accordance with the rotating of the rotary shaft 41 without resistance during the driving of the motor 28.

When the motor 28 stops, as shown in Fig. 3B, applying voltage to the coil portion 42 of the holding brake 30 is stopped. N pole of the pressing plate braking portion 48 pulls the S pole of the pressing plate 44 by the stop of applying voltage to the coil portion 42, the pressing plate 44 is urged toward the pressing plate braking portion 48 side, and the pressing surface 44a is pressed to the pressing plate braking portion 48. The pressing surface 44a of the pressing plate 42 is thus contacted with and pressed to the pressing plate braking portion 48 during the stop of the motor 28, and accordingly the rotary shaft 41 is braked by frictional force in the pressing plate 44a.

When the rotary shaft 41 is braked, as shown in Fig. 2, the male screw shaft portion 34 of the linear motion link device 1 is locked and the inner rod portion 23 is locked, so that the linear motion link device 1 does not slide even if force is applied in the longitudinal direction thereof. Thus, the linear motion link device 1 does not telescope during the stop of the motor 28.

Moreover, in the first embodiment, the holding brake 30 is provided on the rear portion side of the motor 28, however, it is not limited thereto, the holding brake 30 may be provided on the front side of the motor 28.

Further, in the first embodiment, the holding brake 30 presses the pressing plate 44 by magnetic force to generate braking force, however, it is not limited thereto, the holding brake 30 may press the pressing plate by elastic force of an elastic body such as a coil spring or a plate spring, etc., to generate braking force.

Furthermore, in the first embodiment, the linear motion link device 1 is formed to freely telescope in the longitudinal direction thereof by the feed screw mechanism with use of the motor as an actuator, however, it is not limited thereto, the linear motion link device 1 may employ a cylinder employing oil hydraulic, hydraulic or air pressure, etc., or a linear motion link type actuator having a form in which rotational motion is changed to linear motion via a rack pinion, etc., in place of the feed screw mechanism with use of the motor. When the oil hydraulic cylinder is employed, an opening/shutting valve or a servo valve, etc., which stops supplying oil to the cylinder or stops an outflow of oil from the cylinder may be employed as the holding brake. In this case, a timing of opening/shutting of the opening/shutting valve or the servo valve is similar to the timing of the operation of the holding brake explained in the first embodiment, and thus similar effect can be obtained.

The linear motion link device in the first embodiment is constituted as described above, and has operations as described below.
(1) The holding brake 30 can brake rotating of the rotary shaft 41 of the motor 28, and therefore the linear motion link device 1 can stop the motor 28 in a state of supporting a heavy load, the supporting state can be maintained.
(2) When the linear motion link device 1 stands still in the state of supporting the heavy load, the motor 28 can be stopped with the standing still state maintained by the holding brake 30, and therefore a load torque applied to the motor 28 can be reduced, consumption power of the motor 28 can be saved.
(3) Even if the motor suddenly stops by a breakdown, etc., in the state that the linear motion link device 1 supports the heavy load, the holding brake 30 holds and locks the rotary shaft 41, and thus the supporting state can be maintained. In this point, it is excellent in stability and safety.

Next, a bipedal walking robot provided with the linear motion link device explained in the first embodiment will be explained.

### Embodiment 2

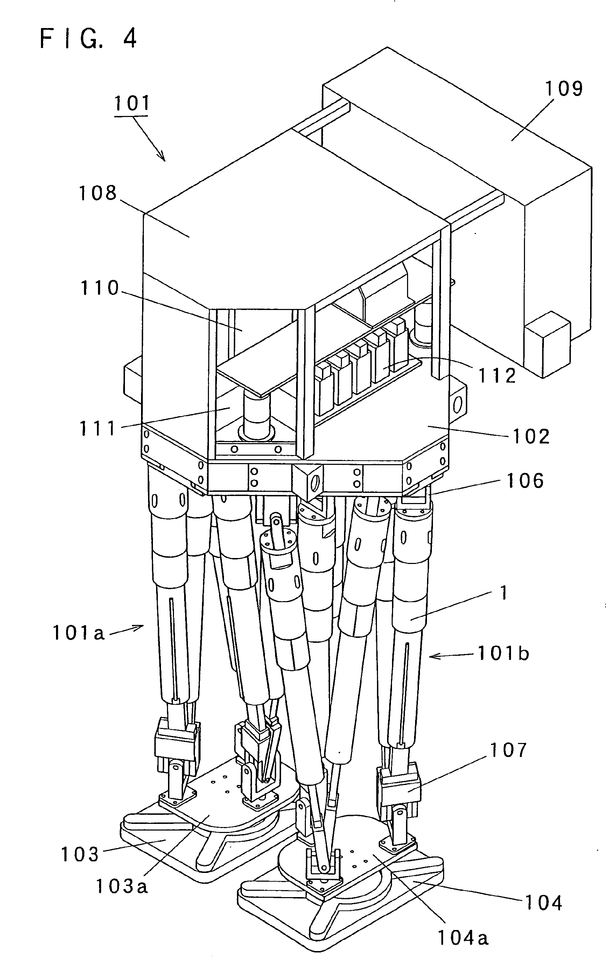

Fig. 4 is a perspective view of a bipedal walking robot of the second embodiment.

In Fig. 4, reference symbol 1 denotes the linear motion link device explained in the first embodiment, 101 denotes the bipedal walking robot of the second embodiment, 101a denotes a parallel link mechanism portion of a right leg of the bipedal walking robot 101, 101b denotes a parallel linkmechanismportion of a left leg of the bipedal walking robot 101, 102 denotes a base, 103 denotes a right foot provided with separation under the base 102, 103a denotes a plane-shaped fixed plate fixed to an upper portion of the right foot 103, 104 denotes a left foot provided in parallel with the right foot 103 with separation under the base 102, 104a denotes a plane-shaped fixed plate fixed to an upper portion of the left foot 104, 106 denotes a base side universal passive joint fixed to a predetermined position under the base 102, 107 denotes foot side universal passive joints which are fixed to predetermined positions above the fixed plate 103a of the right foot 103 and the fixed plate 104a of the left foot 104 respectively, 108 denotes a controller provided on an upper surface of the base 102, 109 denotes a computer for controlling provided on a rear portion of the controller 108, 110 denotes a gyro, 111 denotes a battery and 112 denotes a circuit portion for motor driving.

Here, upper ends of the two linear motion link devices 1 as one set are respectively connected to the base side universal passive joint 106, lower ends of the linear motion link devices 1 are connected to the foot side universal passive joint 107 as one, and as a whole, the linear motion link devices 1 are provided in a V-shape. The three sets of the linear motion link devices 1 are provided in the right legs and the left legs in plane view triangular shape, the six link devices 1 per one leg, and total twelve link devices 1 are provided. That is, the right and left legs of the bipedal walking robot 101 of the first embodiment are respectively constituted by a Stewart platform. Thus, it is excellent in stability of operation and strength. Further, the upper end of one linear motion link device 1 is connected to one base side universal passive joint 106, the lower ends of two linear motion link devices 1 are connected to one foot side universal passive joint 107.

The gyro 110, the battery 111 and the circuit portion for motor driving 112 are provided in the controller 108. In the second embodiment, a nickel-hydrogen battery was employed as the battery 111.

Further, a six-axis force sensor which detects floor reaction force may be provided on the bottom sides of the right foot 103 and the left foot 104. The six-axis force sensor can simultaneously and continuously detect each component of forces of three axis directions and each component of moments of three axis circumference directions with high precision. Furthermore, ZMP (Zero Moment Point) control can be performed based on a value detected by the six-axis force sensor.

The operation of the bipedal walking robot of the second embodiment constituted as described above will be explained below with reference to the drawings. Moreover, in the second embodiment, only the operation of the right leg of the bipedal walking robot 101 will be explained and an explanation regarding the operation of the left leg will be omitted, because the operation of the left leg is similar to the operation of the right leg.

When the right leg is operated, a reverse kinetics of the right foot 103 is calculated based on a predetermined walking pattern, each motor of the linear motion link devices 1 is driven based on the calculated value, the inner rod portion 23 is slid, and the linear motion link device 1 telescopes. The base side universal passive joint 106 provided at a connecting portion of the linear motion link device 1 and the base 102 and the foot side universal passive joint 107 provided at a connecting portion of the linear motion link device 1 and the right foot 103 follow telescopic motion of the linear motion link device 1 without preventing the telescopic motion and are smoothly driven by the linear motion link device 1. Rotating of the motor 28 of the linear motion link device 1 is detected by the rotary encoder 31 provided in the linear motion link device 1, the obtained detected value is fed back as angle data, and the linear motion link device 1 is feed-back controlled. Thus, the right foot 103 can perform operation such as stepping forward by one step or stepping at the position, etc. Further, the right foot 103 and the left foot 104 mutually and continuously perform such operation, whereby walking operation can be performed.

When the bipedal walking robot 101 is in an operating state, power is applied to the motor 28, and the coil portion 42 is also energized. Thus, the holding brake 30 is released from a holding and locking state.

In the case that the bipedal walking robot 101 is in a standing still state, that is, a posture fixed state, when supplying power to the motor 28 is stopped, with which, energizing the coil portion 42 is stopped, the holding brake 30 holds and locks the rotary shaft 41 of the motor 28, and the inner rod portion 23 is locked. Thus, the linear motion link device 1 is not driven by its weight, and does not telescope, and the posture in the standing still state can be maintained.

Moreover, in the second embodiment, an external switch capable of making the holding brake 30 operate or capable of stopping the motor 28 may be provided. Thus, an operator of the bipedal walking robot 101 can speedily stop operation of the bipedal walking robot 101 in the case of perceiving a danger during the operation and the bipedal walking robot 101 can maintain the current posture. Therefore, neither damage of the bipedal walking robot 101 itself by a fall at a sudden stop nor an accident by a fall at the sudden stop occur, and in this point, it is excellent in stability and safety.

The bipedal walking robot in the second embodiment is constituted as described above, and has operations as described below.
(1) In the standing still state, that is, the posture fixed state of the bipedal walking robot 101, the rotary shaft 41 of the motor 28 can be held and locked by the holding brake 30. Therefore, the inner rod portion 23 is not driven by its weight and can maintain the standing still posture, and a load applied to the motor 28 can be reduced.
(2) In the standing still state, that is, the posture fixed state of the bipedal walking robot 101, supplying power to the motor 28 can be stopped, and therefore consumption energy of the motor 28 can be reduced, operating time by exchanging or charging of a mounted battery 111 at one time is extended, and an operating rate of the bipedal walking robot 101 can be improved.
(3) Even if the motor 28 suddenly stops operating by a breakdown, etc., the bipedal walking robot 101 can maintain the current posture. Therefore, neither damage of the bipedal walking robot 101 itself by a fall nor an accident by a fall occur, and in this point, it is excellent in stability and safety.
(4) At the time of transporting or waiting for the bipedal walking robot 101, the current posture of the bipedal walking robot 101 can be maintained without driving of the motor 28, and therefore a load torque applied to the motor 28 can be reduced, and power consumption of the motor 28 can be saved.
(5) The legs are formed by parallel link mechanisms, in which the plurality of linear motion link devices 1 are provided in parallel between the base 102 and the right foot 103 and between the base 102 and the left leg 104, and therefore power of the right foot 103 and the left foot 104 is enlarged, the legs can sustain a large load. Thus, it is possible to transport a heavy load, and in this point, it is excellent in practicality.
(6) The right and left legs of the bipedal walking robot 101 are formed by parallel link mechanisms, in which the plurality of linear motion link devices 1 are provided in parallel between the base 102 and the right foot 103 and between the base 102 and the left leg 104, to sustain a large load, and therefore it is possible to transport a heavy load, and in this point, it is excellent in practicality. Further, compensating operation of an upper half body of the bipedal walking robot 101 is not required for operation of the legs of the robot 101, and therefore it is possible to mount or incorporate the upper half body with a large weight, in this point, it is excellent in the degree of freedom in designing.
(7) A positional error of the right foot 103 or the left foot 104 with errors of movable displacement of the linear motion link devices 1 during the walking operation is averaged, and therefore operation with high precision can be performed. Further, a high resolution is not required for the rotary encoder 31 which detects the displacement of the linear motion link devices 1, and therefore it is excellent in productivity.
(8) Only expansion force and pressure force are applied to each linear motion link device 1, a bending moment is not applied to the link device 1, and therefore it is excellent in strength and rigidity, and excellent in the degree of freedom in designing in the point that a selection range of materials or shapes is widened. Further, the inner rod portion 23 can be slid by the feed screw mechanism employing the male screw shaft portion 34 and the female screw nut portion 35, and therefore controlling can be easily performed and it is excellent in strength and rigidity.
(9) The plurality of the linear motion link devices 1 may employ the same structure, and therefore it is excellent in cost savings and power savings during the manufacture and maintenance can be improved.
(10) The right and left legs of the bipedal walking robot are respectively constituted by a Stewart platform, in which the two linear motion link devices 1 as one set are provided in a V-shape, and at least three sets of the linear motion link devices 1 are provided in the right foot and the left foot in plane view triangular shape, so that it is excellent in stability and strength.

### Industrial Applicability

As described above, according to a linear motion link device and a bipedal walking robot provided therewith of the present invention, advantageous effects as described below can be obtained.

The invention according to the first aspect of the invention can provide a linear motion link device, in which
(1) even if the actuator is stopped in a state that a heavy load is supported by the linear motion link device, the movable portion can be held and locked by the holding brake. Therefore, the movable portion is not driven by a weight of the heavy load, the supporting state can be maintained.
(2) The actuator can be stopped in the state that the heavy load is supported by the linear motion link device, and therefore a load applied to the actuator can be reduced, and consumption of operating energy of the actuator can be saved.

In addition to the effects of the first aspect, the invention according to the second aspect of the invention can provide a linear motion link device, in which
(1) even if the motor suddenly stops by a breakdown, etc., in the state that the heavy load is supported by the linear motion link device, the supporting state can be maintained by holding and locking the movable portion with use of the holding brake, and therefore it is excellent in stability and safety.

In addition to the effects of the second aspect, the invention according to the third aspect of the invention can provide a linear motion link device, in which
(1) the inner rod portion can be locked by braking rotating of the rotary shaft with use of the holding brake, and therefore the motor can be stopped in the state that the heavy load is supported by the linear motion link device, and the supporting state can be maintained.
(2) When the linear motion link device stands still in the state of supporting the heavy load, the motor can be stopped with the standing still state maintained by the holding brake, and therefore a load torque applied to the motor can be reduced, and consumption power of the motor can be saved.

The invention according to the forth aspect of the invention can provide a bipedal walking robot, in which
(1) in a standing still state, that is, a posture fixed state of a bipedal walking robot, the movable portion of the actuator can be held and locked by the holding brake, and therefore, the movable portion of the actuator is not driven by a weight of the robot itself, the standing still state can be maintained, and a load applied to the actuator can be reduced.
(2) In the standing still state, that is, the posture fixed state of the bipedal walking robot, supplying operation energy to the actuator can be stopped, so that consumption energy of the actuator can be reduced, and, for example, when power is supplied to the actuator by a mounted battery, operating time by exchanging or charging of the battery at one time is extended, and an operating rate of the bipedal walking robot can be improved.
(3) Even if the actuator suddenly stops by a breakdown, etc., during the operation, the bipedal walking robot can maintain the current posture, and therefore, neither damage of the bipedal walking robot itself by a fall nor accident by a fall occur, and in this point, it is excellent in stability and safety.
(4) At the time of transporting or wai ting for the bipedal walking robot, the current posture of the bipedal walking robot can be maintained without driving of the actuator, and therefore the load torque applied to the actuator can be reduced, and consumption of operating energy of the actuator can be saved.

In addition to the effects of the forth aspect, the invention according to the fifth aspect of the invention can provide a bipedal walking robot, in which
(1) each parallel link mechanism portion employed in the right and left legs is constituted by a Stewart platform, in which the two links as one set are provided in a V-shape and the three sets are provided, and therefore it is excellent in stability and rigidity, and operation controlling can be simplified.

## Claims

1. A linear motion link device comprising an actuator and a holding brake which holds and locks a movable portion of said actuator, and freely telescopes in a longitudinal direction of the linear motion link device by driving of said actuator.

2. The linear motion link device according to Claim 1, wherein said holding brake holds and locks said movable portion of said actuator at a stop of said actuator.

3. The linear motion link device according to Claim 1 or 2, further comprising:
a motor as said actuator;
a long bar-shaped male screw shaft portion fixed to a rotary shaft of said motor and provided in the longitudinal direction of said linear motion link device;
a female screw nut portion screw-engaged with said male screw shaft portion;
an inner rod portion which is fixed to said female screw nut portion and slides in the longitudinal direction of said linear motion link device by driving of said motor; and
a holding brake which holds and locks said rotary shaft of said motor.

4. A bipedal walking robot comprising:
a base;
a right foot and a left foot;
a plurality of passive joints respectively provided on said base, said right foot and said left foot;
parallel link mechanism portions respectively provided between said passive joints provided on said base and said passive joints provided on said right foot and between said passive joints provided on said base and said passive joints provided on said left foot; and
the linear motion link devices according to any one of Claims 1 to 3 as a link of said parallel link mechanism portions.

5. The bipedal walking robot according to Claim 4, further comprising:
the respective three sets of said parallel link mechanism portions which are provided between said base and said right foot and between said base and said left foot.
